Europäisches Patentamt

European Patent Office

Office européen des brevets

(19)

(11) Publication number: **0 251 656**

**A2**

(12) # EUROPEAN PATENT APPLICATION

(21) Application number: **87305578.4**

(22) Date of filing: **23.06.87**

(51) Int. Cl.⁴: **A63B 23/04**

(30) Priority: **23.06.86 US 877117**

(43) Date of publication of application:
**07.01.88 Bulletin 88/01**

(84) Designated Contracting States:
**AT BE CH DE ES FR GB GR IT LI LU NL SE**

(71) Applicant: **LOREDAN BIOMEDICAL, INC.**
**1632 Da Vinci**
**Davis California 95616(US)**

(72) Inventor: **Bond, Malcolm L.**
**718 Hemenway Street**
**Winters California 95694(US)**
Inventor: **Dempster, Philip T.**
**326 Hidalgo Place**
**Davis California 95616(US)**

(74) Representative: **Cross, Rupert Edward Blount**
**et al**
**BOULT, WADE & TENNANT 27 Furnival Street**
**London EC4A 1PQ(GB)**

(54) **Apparatus for diagnosis and/or training of proprioceptor feedback capabilities in a muscle and joint system of a human patient.**

(57) An apparatus for diagnosis and/or training of proprioceptor feedback capabilities of a muscle and joint system of a human patient using an exercise system having a patient attachment device and an arrangement for controlling parameters of an exercise movement in response to a control signal derived from one or more measured exercise parameters. A patient performance goal is defined as a real time function of preselected exercise parameters. A perturbation signal function is defined for the control signal; and the control signal is modified by the perturbation function value during a patient exercise motion. The defined performance goal is displayed during the patient exercise motion and the actual patient performance relative to the performance goal is also tracked and displayed. An error value is measured as the difference between actual patient performance and the patient performance goal.

FIG. — 4

# APPARATUS FOR DIAGNOSIS AND/OR TRAINING OF PROPRIOCEPTOR FEEDBACK CAPABILITIES IN A MUSCLE AND JOINT SYSTEM OF A HUMAN PATIENT

This invention relates generally to exercise systems and training systems and, more specifically, to an apparatus for diagnosis and/or training of proprioceptor feedback capabilities of a muscle and joint system of a human patient using an exercise system having a patient attachment device and an arrangement for controlling parameters of an exercise movement in response to a control signal.

Proprioception of human joints involves neural mechano-receptors associated with the joint, tendon and spindle receptors in the muscle associated with joint movement. These receptors have been categorized as either position sense receptors or dynamic sensing receptors, i.e. for sensing velocity of movement. A number of mechanisms have been proposed as being responsible for proprioceptive sensing. One theory is that the joint mechano-receptors project their output directly onto the muscle spindle system and set the gain or threshold of the spindle nerve fibers response to muscle stretch. The perception of stretch to the muscle when the muscle spindle system is at high gain shortens the time required for coordinated muscle contractile response and produces greater motor control in a particluar movement. Any stress on the mechano-receptors in the joint capsule is relayed to the spindle system, which in turn adjusts the muscle tone to suit the situation.

Injury to a human extremity may be associated with direct damage to muscle tissue. Artificial immobilization during biological repair of muscle tissue may produce pathological disuse atrophy of the muscle tissue. Conventional rehabilitation of the extremity after injury and healing usually involves diagnosing and retraining the functionality of the involved joint with the status of rehabilitation inferred from parameters such as range of joint motion, attainment of normal muscular strength and normal fatigue resistance. However, even after rehabilitation with reference to these parameters, it is likely that subtle differences still remain in the peripheral neurosensory organs in the involved joint and related musculature. Deficiencies in the performance of these neurosensory organs affect the proprioceptive feedback system which is used to produce coordinated movement of the joint. the implications of such remaining damage after rehabilitation are important, particularly in normal human gait activities (e.g., walking and running) and specialized movements in sports activity where proprioception plays a fundamental role in the quantitative control, organization and timing of body action. It is believed that remaining damage in peripheral neurosensory organs may be reponsible for cases of repetitive injury. In other words, although strength of the joint and muscle associate therewith may have been rehabilitated, remaining damage to the proprioceptive feedback mechanisms may preclude sufficiently coordinated movement to avoid reinjury during either normal human activity or specialized sports movements.

In the case of direct injury to the joint mechano receptors, as sometimes occurs in joint sprains. the patient is unable to exhibit coordinated movement about the joint during a weight bearing exercise event. the diagnosis of this condition appears to be that the muscle spindles are deficient in sensory input due to the damage to the mechano receptors in the articular structures associated with the joint.

Very little research has been done to evaluate proprioception integrity. One test that has been developed for evaluation of proprioception integrity is to analyze and grade the response of the muscles engaged in controlled movement to the sudden application of a resistance force. In one research project the application of resistance was provided by a breakaway trip wire attached to the foot of the patient. Electromyographic studies of the muscles provided evaluation capability for determining the muscular response. The graded score of proprioception integrity was determined by the latency time between the application of resistance and the muscular response to return to a normal gait movement. The shorter the time, the tighter the neural control of position and velocity duplication ability of the involved joint and associated muscles.

It is the principal object of this invention to provide an apparatus for diagnosis and/or training of proprioceptor feedback capabilities of a muscle and joint system of a human patient using a controllable exercise system which can provide a variety perturbation signals in one or more exercise control parameters associated with the system.

More specifically, it is an object of this invention to provide an apparatus for diagnosis and/or training of proprioceptor feedback capabilities in a muscle and joint system using a controllable resistance exercise system.

It is another object of this invention to provide a methodological capability for assessing the effectiveness of controllable exercise systems in diagnosis of proprioceptive feedback integrity.

In its broadest aspects, this invention features an apparatus for diagnosis and/or training of proprioceptor feedback capabilities of a muscle and joint system of a human patient using an exercise system having a patient attachment device together with means for controlling parameters of an exercise movement in response to a control signal derived from one or more measured exercise parameters. The apparatus further comprises means defining a patient performance goal as a real time function of preselected exercise parameters. A perturbation signal function is defined for the control signal by further means and preferably that perturbation signal furnction applies regular unexpected changes in the exercise system control signal so that the system can determine the adaptive response capability associated with the integrity of the proprioceptive neurosensory feedback loop in the body. The apparatus also includes means modifying the control signal by the perturbation function value during a patient exercise motion. To implement the biofeedback aspect, the defined performance goal is displayed during the patient exercise motion and the actual patient performance relative to that goal is also displayed during the patient exercise motion. An error value is measured as the difference between actual patient performance and the patient performance goal during the patient exercise motion.

The advantage of the apparatus of this invention is that it permits quantification of the proprioception neurosensory feedback integrity. It is also expected that this apparatus will provide for retraining of these neurogenic components of muscle contraction as a separate aspect of a patient rehabilitation program. Since this is a field of research and development which is in its infancy, the main initial value and advantages of this apparatus lies in its use as a research tool. the degree of effectiveness of diagnosis and training of proprioceptor feedback capabilities will await extensive research studies. However, the current foundation of knowledge regarding proprioceptor feedback and the apparatus of this invention provide a reasonable basis for concluding that at least some system implementations of this apparatus have a strong probability of producing very significant diagnostic capabilities and proprioceptive feedback retraining capabilities.

Fig. 1 is an isometric drawing of an isokinetic station comprising an exercise system in accordance with the present invention.

Fig. 2 is a general block diagram of exercise system components in accordance with the present invention.

Fig. 3 is a generalized flow diagram setting forth the basic steps carried out by an apparatus in accordance with this invention.

Fig. 4 is a block diagram of a particular form of digital real time control exercise system in accordance with the present invention.

Fig. 5 is a pictorial display of a sample feedback display aspect of one embodiment of this invention.

Figs. 6 and 7 illustrate generalized software routines which may be implemented in the system of Fig. 4 in specific embodiments of the apparatus in accordance with the present invention.

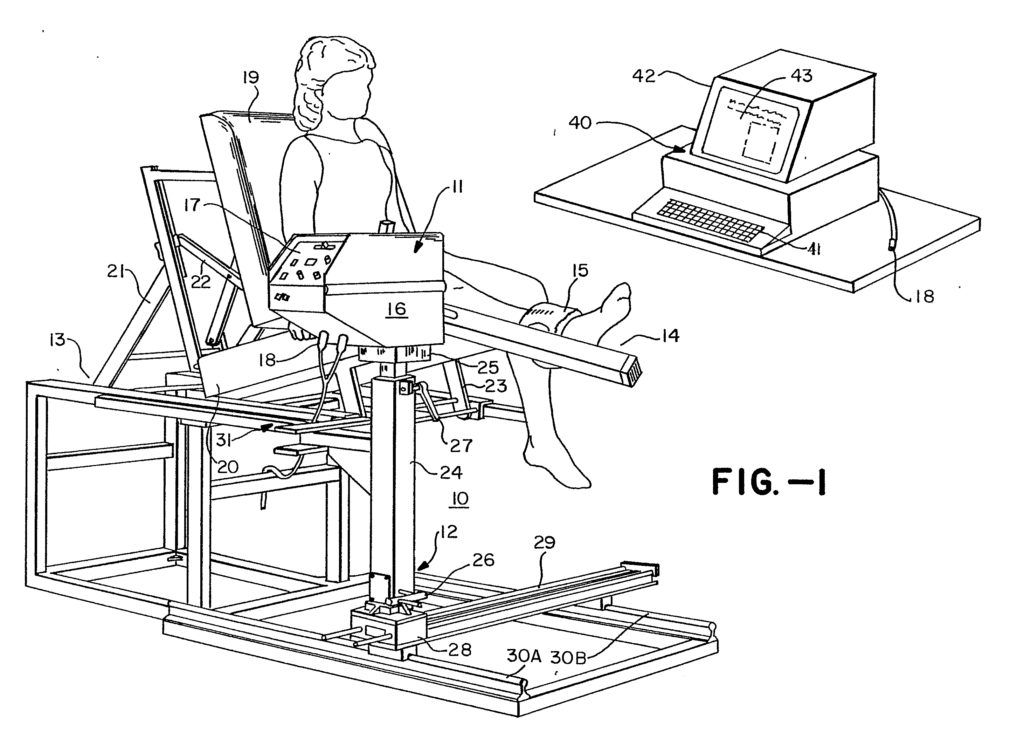

The apparatus may comprise an isokinetic exercise system of the type shown in Fig. 1 as well as a variety of other exercise systems. The isokinetic station of Fig. 1 is a whole body system which has been designed for maximum utility in patient positioning, optimum flexibility in set up for exercise of various portions of the human body, and minimum involved floor space.

Referring to Fig. 1, isokinetic station 10 includes a passive exercise resistance system 11, a mounting arrangement 12 and a patient couch 13. The passive resistance system 11 includes a lever arm assembly 14, a patient attachment cuff 15, a housing 16 which contains the passive resistance component of the system along with electronic controls. The housing 16 further includes a control panel 17 and output leads 18 which feed measurement signal outputs to a chart recorder and/or to a computer data acquisition unit 40. the details of this passive exercise resistance control system will be given below in connection with other drawing figures.

The patient couch arrangement 13 includes two cushion portions 19 and 20 which, together with various positioning elements, provide for positioning of a patient in a sitting or reclining orientation, which is selected depending on the patient limb being exercised. In the set-up shown in Fig. 1, the cushion portion 19 is serving as a backrest and the cushion portion 20 is serving as a seat. A pair of positioning members 21 controls the angular orientation of the cushion portion 19 and a scissors jack type of positioning arrangement 22 controls the forward and backward position of the cushion portion 19.

Positioning supports 23 control the angle of the cushion element 20. To put the patient in a reclining position, the positioning members 23 and 21 are reoriented so that the cushion elements 19 and 20 are horizontal and in line with each other.

The mounting and positioning system 12 includes a vertical pedestal arrangement 24 which includes a rotary support member 25 to which the housing of the passive resistance system 16 is attached. Preferably a detent arrangement is provided such that the angular orientation of the housing 16 relative to the patient couch can be selectively altered to fixed angles. A height adjustment jacking arrangement operated by the jack handle

5    0 251 656    6

26 is provided within the pedestal 24 to raise and lower the housing 16 for positioning of the axis of rotation of the lever arm assembly 14 relative to the patient.

The pedestal assembly 24 is mounted on a bearing slide arrangement 28 which permits side-to-side movement of the pedestal assembly 24 relative to the patient couch. Another bearing and track arrangement 30A and 30B permits front-to-back movement of the pedestal 24 carried on the bearing and track arrangement 28 and 29. A stabilizing arrangement 31 is provided to rigidly fix the pedestal 24 in a particular selected position relative to the patient couch assembly 13.

The remote computer system 40 includes a keyboard 41, and a display device 42 having a display screen 43. this remote computer system carries out the software routines associated with an aspect of this invention based on data that is acquired from the real time control computer in the exercise control unit 16 and on control signals that are sent back to the real time control computer. The display screen 43 displays to the patient exercising on the system the current value of the exercise parameter associated with a preselected exercise goal and the actual parameter value which is being attained by the patient. The details of this display will be discussed in more detail below.

Fig. 2 illustrates the basic components of an exercise system in accordance with an embodiment of the present invention. The required system features includes a patient attachment arrangement 50 and a controllable movement system 51 which may be one or both of an active or passive system. A torque or force measuring system 52 and a position measuring system 53 determine exercise parameters and feed signals to computer control system 54. Computer control system 54 runs a software program to provide an exercise control signal to the controllable movement system 51. Computer control system 54 also provides an output to a patient feedback display on which both the exercise goal and the actual value of exercise parameter may be displayed back to the patient.

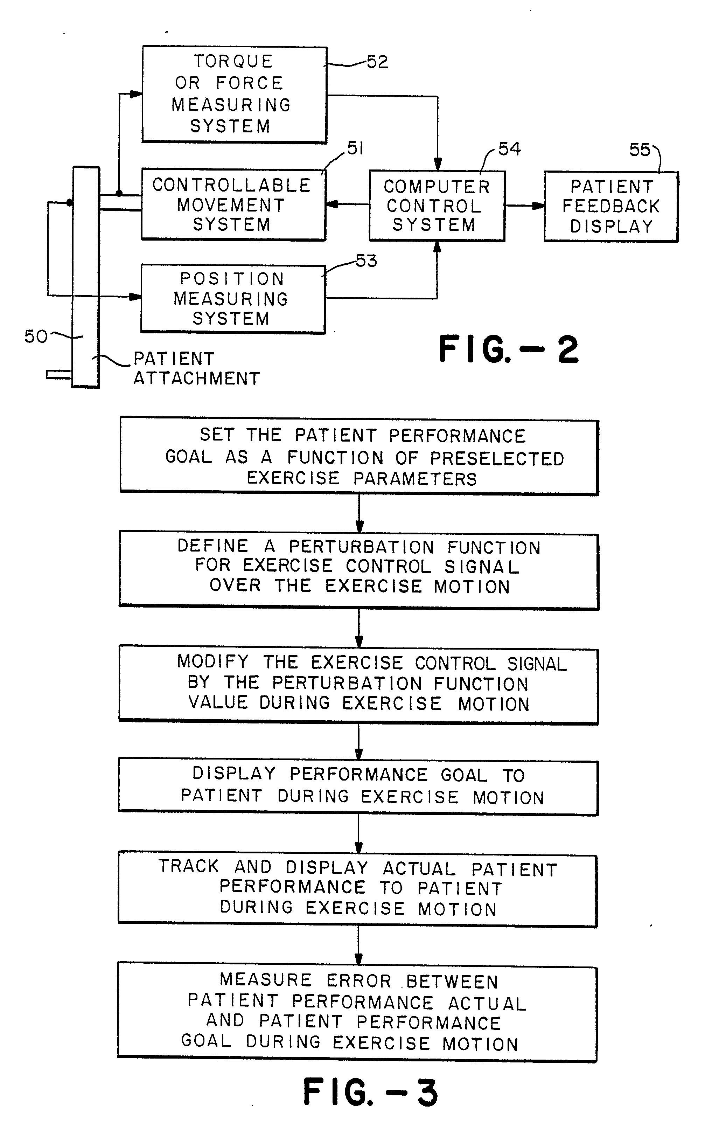

Fig. 3 illustrates the basic steps carried out by an apparatus for diagnosis and/or training of proprioceptor feedback capabilities in accordance with the present invention. The first step carried out by the apparatus involves defining a patient performance goal as a function of preselected exercise parameters. This patient performance goal may, for example, be related to position of the patient attachment system as a function of time, force or torque on the system as a function of position or some other parameter. Other possibilities for patient performance goals are such things as applied power as a function of position and the like.

A following step carried out by the apparatus is defining a perturbation function for the exercise control signal over the exercise motion. It is preferable that the perturbation function be one that alters the exercise control signal in at least a semi-random manner so that the perturbations experienced by the exercising patient are not predictable in time or both time and amplitude.

The apparatus also comprises means modifying the exercise control signal by the perturbation function value during the exercise motion. This is preferably done on a proportional basis so that the exercise control signal does not move out of the range of the patient's ability to adapt to the perturbation based on the patient's strength.

Further features of the apparatus are means to display the defined patient performance goal to the patient during the exercise motion along with means to track and display actual patient performance relative to the defined goal during the exercise motion. In this manner, the patient is able to utilize visual biofeedback to attempt to track the displayed performance goal despite the perturbations being introduced into the exercise control signal.

Another important feature of the apparatus is means to measure the error between the patient performance actual and patient performance goal during the exercise motion. Assuming serious and determined attempts by the patient to match performance of the exercise motion to the displayed performance goal, the error measurements should provide an indication of proprioceptor feedback integrity of the muscles and joint involved in the exercise motion. It should be appreciated that wide variety of qualitative and quantitative error analysis methods can be experimented with to assess the ability of the apparatus of this invention to quantitate the integrity of the proprioceptor feedback capabilities of the patient. The error analysis can include such simple analysis algorithms as cumulating error measurements over selected portions of the exercise motion. The error analysis may also include sophisticated Fourier analysis of the spectral content of the error signal which may eventually provide information on the type and degree of proprioceptor feedback deficiency.

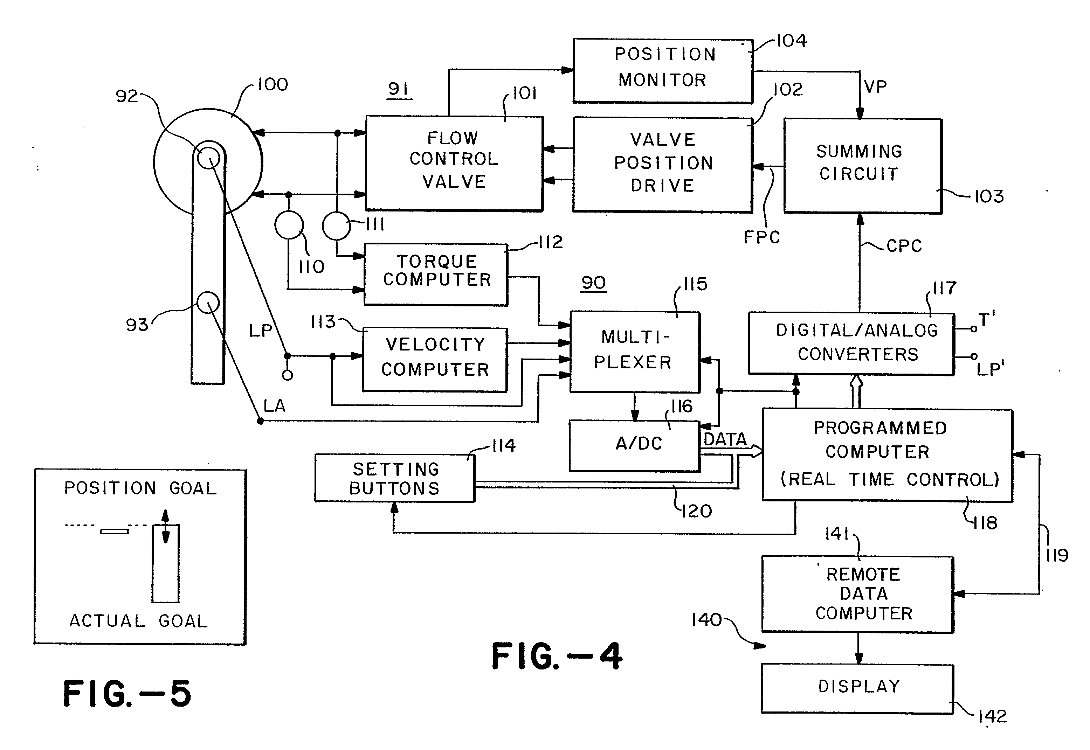

Fig. 4 is a block diagram which illustrates a digital signal processing version of a velocity control computer 90 in accordance with this invention. A torque computer 112 and a velocity computer 113 have their outputs coupled into multiplexer 115 along with the actual limb length signal LA and the lever position signal LP. The setting buttons 114 provide data on a dataline 120 under programmed computer control. Multiplexer 115 functions under the control of programmed computer 118 to multiplex one of the input analog signals to the analog

4

to digital converter 116. Analog to digital converter 116 functions under computer control to convert the analog signal at its input to a digital data signal for input to the programmed computer 118. Digital to analog converters 117 receive output data from the programmed computer 118 and function under its control to provide output analog signals. One of these output analog signals is the compensated position control signal CPC. The other two output signals are torque T' and lever position LP' representing correct values for these parameters.

The programmed computer 118 which provides real time control of the exercise system is couple via a data link 119 to a remote data computer system 10. The remote data computer system 140 includes a programmable computer 141 that executes software routines of this system embodiment. A display device 142 coupled to the computer 141 provides one form of the patient feedback display capability in accordance with the present invention.

It is seen from Fig. 4 that there are two analog computer circuit systems remaining in the velocity control computer system in this particular implementation. The torque computer 112 and the velocity computer 113 are conveniently implemented in analog computer circuitry to reduce the number of digital inputs that have to be taken into the programmed computer. It should be understood that the torque computation could be accomplished by separately multiplexing and converting the outputs of the pressure transducer subsystems 110 and 111. It should also be understood that the velocity computation could be made by the programmed computer instead of utilizing an analog version of that computational function.

The details of a specific embodiment of real time control software being executed in the programmed computer 118 is given in a co-pending and commonly assigned Bond et al. patent application U.S. Serial No. 845,861, filed March 28, 1986. The passive, isokinetic exercise system depicted in Fig. 4 is not the only type of system which may be used in an apparatus according to the present invention. Generally, the apparatus may comprise any exercise system, active or passive, in which electronic control over the basic system parameters affecting the movement of the system by the patient is provided.

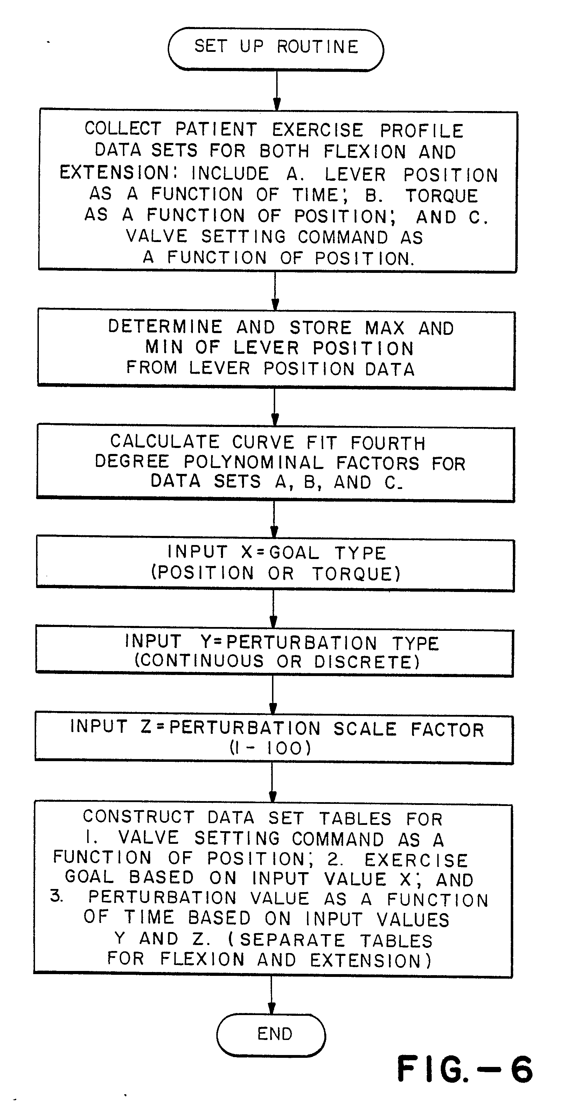

Fig. 5 illustrates a type of patient feedback display that may be used in connection with this invention. a position versus time goal is displayed with the goal bar moving up and down to indicate the goal of the lever position relative to time after turnaround from the maximum extension and flexion of the exercise movement. The display of the actual position shows the patient the actual lever position relative to the goal so that the patient can try to track the goal position as accurately as possible. In the algorithm implemented in one version of this invention, the goal display displays position as a function of time but only begins to change in a positive or negative direction after the patient reaches the actual limit of exercise motion and turns around with sufficient application of torque on the lever arm to signal a turnaround. It should be apparent that other algorithms for displaying the patient position goal could be implemented.

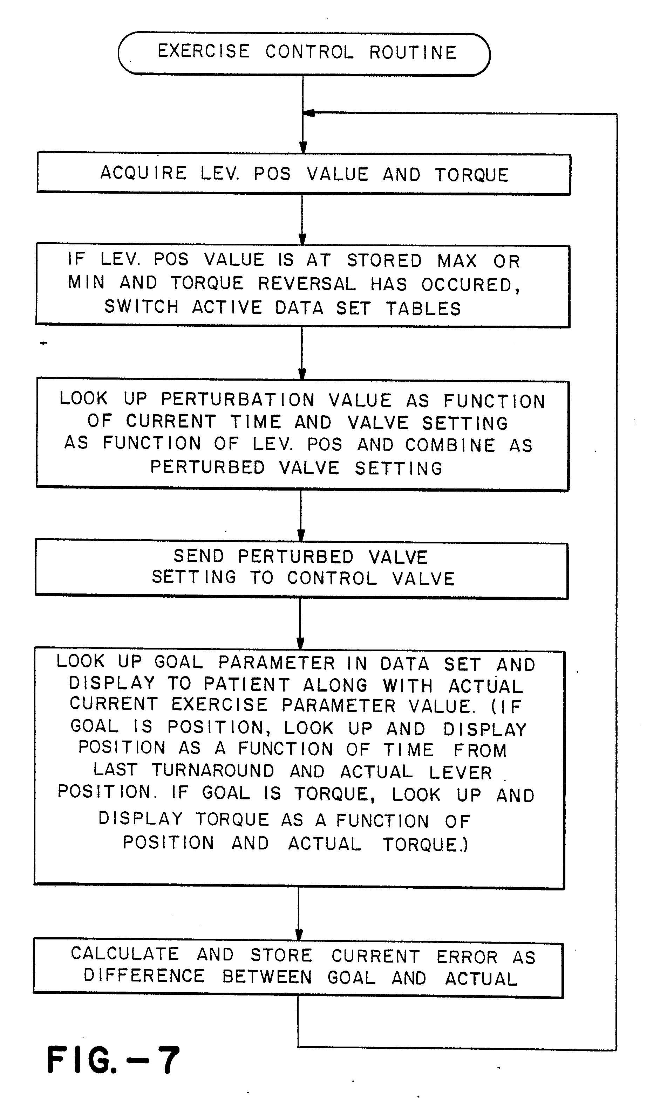

Figs. 6 and 7 illustrate the general steps of a set-up routine and an exercise control routine in accordance with one embodiment of the present invention as implemented on the system of Fig. 4. These routines are carried out in the remote data computer 141 in the case of the system of Fig. 4. It should be understood however that, in other systems configurations it may be possible to combine the functions of the real time control computer 118 and the remote data computer 141 into a single computer control and data analysis system.

The set-up routine depicted in Fig. 6 is implemented in an embodiment of this invention in which the patient performance goal is defined on a basis of patient exercise profile data which is collected during several repetitions of the exercise motion on the system. Accordingly, the first general step of this set-up routine involves collecting patient exercise profile data sets for both the flexion and extension movements. These data sets include a data set A comprising lever position as a function of time, a data set B involving torque as a function of position and a data set C involving valve setting command as a function of position. A subsequent step of the routine involves determining and storing the maximum and minimum lever positions of the patient's exercise motion from the lever position data which has been stored. It should also be understood that the MAX and MIN lever position information could be obtained from the upper and lower limit settings which may be set in using the setting buttons 114 shown in Fig. 4. Reference is made to the co-pending patent application referred to above for an explanation of these limits setting functions.

Another step of the routine then involves calculating curve fit fourth degree polynomial factors for the data sets A, B and C referred to above. In this manner a smooth curve is fit to the acuqired data so that, where necessary in later control functions, appropriate data parameters can be obtained over the whole range of data values. These curve fit polynomial factors are stored for later use.

The next steps of the routine involve inputting certain set-up selections such as goal type, perturbation type and perturbation scale factor. In the current implementation of the invention the goal types are selectable to be either a position goal or a torque goal. The position goal involves lever position as a function of time. The torque goal involves torque value as a function of lever position. The perturbation types available in the current implementation involve either a continuous type of perturbation or a discrete perturbation. In the continuous type of perturbation the transition between perturbation values applied to the valve setting command signal (discussed later) provide a relatively smooth change in the amplitude of the perturbation signal. In the discrete case, the perturbation amplitude varies between two values with the time of the change between values being substantially random. The perturbation scale factor is currently set from 1 to 100. This input determines the amplitude of the perturbation applied to the valve setting command.

After these inputs have been received, the routine constructs data set tables for the valve setting command as a function of position, the exercise goal based on input x for goal type and the perturbation value as a function of time based on the input values y and z. Separate data set tables are constructed for flexion and extension since the patient profile data varies for those exercise motions.

The real time control software described in the above-mentioned co-pending patent application utilizes logarithm values for the valve setting command. Accordingly, it is preferred that the valve setting command table and the perturbation value table be structured as logarithm tables so that these values can be added together to achieve automatic proportioning of the perturbation to the degree of resistance which is commanded by the valve setting command.

After these data set tables have been constructed, the patient can begin to exercise on the system while the remote data computer 141 shown in Fig. 4 is running the exercise control routine. It should be understood that during the proprioception feedback exercise approach, the valve setting command which is normally sent to the circuitry which operates the flow control valve is interrupted so that a perturbed valve setting can be sent to the flow control valve under the control of the remote data computer. The valve setting command determined by the programmed computer 118 would provide isokinetic control over the movement of the lever arm which is not desired under this proprioceptive exercise approach.

The exercise control routine shown in Fig. 7 includes acquiring the lever position value and torque value determined by the programmed computer 118. The routine determines whether the lever position value is at the stored maximum or minimum and whether a torque reversal has occurred and switches the active data set table if these joint conditions are satisfied. This provides the system capability to utilize different data set table values during flexion and extension motions of the lever arm of the exercise system. The routine next looks up a perturbation value as a function of the current elapsed time and a valve setting as a function of the lever position and combines these values (summing them as logarithm values) to obtain a perturbed valve setting. It is this perturbed valve control setting which is then sent from the remote data computer 141 back to the real time control computer 118 to be sent to the flow control valve position control circuitry instead of the valve command setting calculated by the real time control computer 118.

The exercise control routine of Fig. 7 also looks up the goal parameter in the appropriate data set and displays this to the patient along with the actual current exercise parameter value. In this manner the patient can determine how close his performance is to the displayed goal and attempt to alter the exercise motion to track the displayed goal. If the goal type selection made in the set-up routine is a position goal the system looks up and displays the position as a function of time from the last turnaround as well as the actual lever position. If the goal is a torque goal, the routine looks up and displays torque as a function of position as well as the actual torque.

Another step of the routine is to calculate and store current error as a difference between the goal and actual, i.e. the difference between the values of these parameters.

In addition to running the exercise control routine shown in Fig. 7, a number of other parameters are acquired by the remote data computer during the exercise routine so that that data can be stored, manipulated, and/or displayed utilizing appropriate program routines.

It should be understood that the set-up and exercise control routines depicted in Figs. 6 and 7 provide a specific implementation of the general concepts of this invention which could readily be altered in many different ways to achieve different specific functionality but remaining within the general concepts of this invention. It should be apparent that once the data has been accumulated for the error values between the exercise goal and actual patient performance, a wide variety of data display and printout routines could be implemented to visually display significant aspects of the data, and perform meaningful data analysis tasks.

While the apparatus this invention has been described above in the form of a general concept and certain specific embodiments, it should be understood that persons of school in the related art of this invention could make numerous modifications and additions without departing from the scope of the invention.

## Claims

1. Apparatus for diagnosis and/or training of proprioceptor feedback capabilities in a muscle and joint system of a human patient comprising controllable resistance exercise means having a resistance value determined by a resistance control signal;
means to define a patient performance goal as a real time function of preselected exercise parameters;
means to define a perturbation function for controllable resistance over the extent of the exercise motion;
means to modify the resistance control signal by the perturbation function value during the exercise motion;
means to display said defined performance goal during a patient exercise motion;
means to track and display actual patient performance relative to said performance goal during a patient exercise motion; and
means to measure as an error value the difference between actual patient performance and said patient performance goal during the exercise motion.

2. Apparatus as claimed in Claim 1, wherein said means to define a patient performance goal comprises means to collect data on one or more preselected exercise parameters during an actual exercise motion by the patient and means to define said patient performance goal as a function of said collected data.

3. Apparatus for diagnosis and/or training of proprioceptor feedback capabilities in a muscle and joint system of a human patient comprising a controllable resistance exercise means having a patient attachment device mounted for movement through space, a resistive component coupled to said patient attachment device to resist the movement of said patient attachment device with a resistance value determined by a resistance control signal, means for tracking position of said attachment, and means for tracking force applied to said attachment, and further comprising;
means to profile the parameters of actual patient exercise movements by collecting data on position of said attachment device as a function of time, force on said attachment device as a function of position, and the value of said resistance control signal as a function of position;
means to produce patient exercise profile curves from said collected data and store representations of said profile curves;
means to derive from said stored curve representations a patient goal performance function selected as one of position as a function of time and torque as a function of position;
means to define a perturbation function for said stored curve representation for said resistance control signal as a function of time;
means to apply during a patient exercise motion a resistance control signal value as a function of actual position derived from said patient exercise profile curves modified by the current value of said perturbation function;
means to display said defined performance goal during a patient exercise motion;
means to track and display actual patient performance relative to said performance goal during a patient exercise motion; and
means to measure as an error value the difference between actual patient performance and said patient performance goal during the exercise motion.

4. Apparatus for diagnosis and/or training of proprioceptor feedback capabilities of a muscle and joint system of a human patient using an exercise means having a patient attachment device and means for controlling parameters of an exercise movement in response to a control signal derived from one or more measured exercise parameters, comprising;
means to define a patient performance goal as a real time function of preselected exercise parameters;
means to define a perturbation signal function for said control signal;
means to modify said control signal by said perturbation function value during a patient exercise motion;
means to display said defined performance goal during said patient exercise motion;
means to track and display actual patient performance relative to said performance goal during a patient exercise motion, and
means to measure as an error value the difference between actual patient performance and said patient performance goal during said patient exercise motion.

5. Apparatus for diagnosis and/or training of proprioceptor feedback capabilities of a muscle and joint system of a human patient comprising a controllable resistance exercise system having an hydraulic actuator with complementary hydraulic fluid chambers and a shaft, a lever arm coupled to said actuator shaft and a flow control valve coupled to said actuator for controlling the degree of resistance applied to said lever arm by said actuator in response to a valve position command signal, means for measuring the lever position and means for measuring the torque applied to said shaft, and further comprising;

means to collect and store patient exercise profile data during a series of actual patient exercises on the system for both flexion and extension exercise movements, including means to collect and store data sets comprising lever position as a function of time from the last turnaround of the lever, torque as a function of lever position, and valve position command signal as a function of lever position;

means to determine from said stored lever position data a maximum and minimum lever position for said profiled patient exercise;

means to calculate curve fit polynomial factors for said stored data exercise;

means to receive and store input goal type, perturbation type, and perturbation scale factor selections;

means to construct and store for each of flexion and extension exercise movements a set of data tables comprising valve setting command values as a function of lever position, exercise goal values based on input goal type, and perturbation values based on input perturbation type and scale factor;

means to receive and store values of current lever position and torque;

means to determine an active data set from current lever position and torque values;

means to select a perturbation value from said data table of perturbation values based on a time factor;

means to select an unperturbed valve setting command from said data table based on current lever position;

means to determine a perturbed valve setting command by combining said unperturbed valve setting command with said perturbation value;

means to communicate said perturbed valve setting command to said flow control valve to set the current value of exercise resistance;

means to determine the current goal parameter value in said goal data set;

means to display said current goal parameter value and the associated current exercise value; and

means to determine and store a current error value based on the difference between said current goal parameter value and said associated current exercise value.

FIG. —1

0 251 656

**FIG.—2**

- TORQUE OR FORCE MEASURING SYSTEM — 52
- CONTROLLABLE MOVEMENT SYSTEM — 51
- POSITION MEASURING SYSTEM — 53
- COMPUTER CONTROL SYSTEM — 54
- PATIENT FEEDBACK DISPLAY — 55
- PATIENT ATTACHMENT — 50

**FIG.—3**

SET THE PATIENT PERFORMANCE GOAL AS A FUNCTION OF PRESELECTED EXERCISE PARAMETERS

DEFINE A PERTURBATION FUNCTION FOR EXERCISE CONTROL SIGNAL OVER THE EXERCISE MOTION

MODIFY THE EXERCISE CONTROL SIGNAL BY THE PERTURBATION FUNCTION VALUE DURING EXERCISE MOTION

DISPLAY PERFORMANCE GOAL TO PATIENT DURING EXERCISE MOTION

TRACK AND DISPLAY ACTUAL PATIENT PERFORMANCE TO PATIENT DURING EXERCISE MOTION

MEASURE ERROR BETWEEN PATIENT PERFORMANCE ACTUAL AND PATIENT PERFORMANCE GOAL DURING EXERCISE MOTION

FIG.-4

FIG.-5

```
                    ┌─────────────────────┐
                    │   SET UP ROUTINE    │
                    └─────────────────────┘
                               │
                               ▼
        ┌──────────────────────────────────────────┐
        │   COLLECT PATIENT EXERCISE PROFILE        │
        │   DATA SETS FOR BOTH FLEXION AND          │
        │   EXTENSION: INCLUDE A. LEVER POSITION     │
        │   AS A FUNCTION OF TIME; B. TORQUE         │
        │   AS A FUNCTION OF POSITION; AND C.        │
        │   VALVE SETTING COMMAND AS                 │
        │   A FUNCTION OF POSITION.                  │
        └──────────────────────────────────────────┘
                               │
                               ▼
        ┌──────────────────────────────────────────┐
        │   DETERMINE AND STORE MAX AND             │
        │   MIN OF LEVER POSITION                    │
        │   FROM LEVER POSITION DATA                 │
        └──────────────────────────────────────────┘
                               │
                               ▼
        ┌──────────────────────────────────────────┐
        │   CALCULATE CURVE FIT FOURTH              │
        │   DEGREE POLYNOMINAL FACTORS FOR           │
        │   DATA SETS A, B, AND C.                   │
        └──────────────────────────────────────────┘
                               │
                               ▼
        ┌──────────────────────────────────────────┐
        │   INPUT X = GOAL TYPE                      │
        │   (POSITION OR TORQUE)                     │
        └──────────────────────────────────────────┘
                               │
                               ▼
        ┌──────────────────────────────────────────┐
        │   INPUT Y = PERTURBATION TYPE             │
        │   (CONTINUOUS OR DISCRETE)                 │
        └──────────────────────────────────────────┘
                               │
                               ▼
        ┌──────────────────────────────────────────┐
        │   INPUT Z = PERTURBATION SCALE FACTOR     │
        │   (1 - 100)                                │
        └──────────────────────────────────────────┘
                               │
                               ▼
        ┌──────────────────────────────────────────┐
        │   CONSTRUCT DATA SET TABLES FOR           │
        │   1. VALVE SETTING COMMAND AS A            │
        │   FUNCTION OF POSITION; 2. EXERCISE        │
        │   GOAL BASED ON INPUT VALUE X; AND         │
        │   3. PERTURBATION VALUE AS A FUNCTION      │
        │   OF TIME BASED ON INPUT VALUES            │
        │   Y AND Z. (SEPARATE TABLES                │
        │   FOR FLEXION AND EXTENSION)               │
        └──────────────────────────────────────────┘
                               │
                               ▼
                    ┌─────────────────────┐
                    │        END          │
                    └─────────────────────┘
```

FIG.—6

EXERCISE CONTROL ROUTINE

ACQUIRE LEV. POS VALUE AND TORQUE

IF LEV. POS VALUE IS AT STORED MAX OR MIN AND TORQUE REVERSAL HAS OCCURED, SWITCH ACTIVE DATA SET TABLES

LOOK UP PERTURBATION VALUE AS FUNCTION OF CURRENT TIME AND VALVE SETTING AS FUNCTION OF LEV. POS AND COMBINE AS PERTURBED VALVE SETTING

SEND PERTURBED VALVE SETTING TO CONTROL VALVE

LOOK UP GOAL PARAMETER IN DATA SET AND DISPLAY TO PATIENT ALONG WITH ACTUAL CURRENT EXERCISE PARAMETER VALUE. (IF GOAL IS POSITION, LOOK UP AND DISPLAY POSITION AS A FUNCTION OF TIME FROM LAST TURNAROUND AND ACTUAL LEVER POSITION. IF GOAL IS TORQUE, LOOK UP AND DISPLAY TORQUE AS A FUNCTION OF POSITION AND ACTUAL TORQUE.)

CALCULATE AND STORE CURRENT ERROR AS DIFFERENCE BETWEEN GOAL AND ACTUAL

# FIG.−7